# EUROPÄISCHE PATENTSCHRIFT

(11) **EP 0 546 388 B1**
(45) Veröffentlichungstag und Bekanntmachung des Hinweises auf die Patenterteilung: **27.03.1996**
(21) Anmeldenummer: 92120187.7
(22) Anmeldetag: 26.11.1992
(51) Int. Cl.: C07D 405/14, C07D 405/06, A61K 31/445, A61K 31/40

(54) **Azaheterocyclylmethyl-chromane als Wirkstoffe zur Behandlung von Erkrankungen des Zentralnervensystems**
Azaheterocyclylmethyl-chromans as agents for the treatment of diseases of the central nervous system
Azahétérocyclylméthyl-chromanes comme agents pour le traitement de maladies du système nerveux central

(30) Priorität: 09.12.1991 DE 4140540
(43) Veröffentlichungstag der Anmeldung: 16.06.1993
(73) Patentinhaber: BAYER AG, D-51368 Leverkusen (DE)
(72) Erfinder: Heine, Hans-Georg, Dr., W-4150 Krefeld (DE); Schohe-Loop, Rudolf, Dr., W-5600 Wuppertal (DE); Glaser, Thomas, Dr. Dr., W-5063 Overath 3 (DE); De Vry, Jean Marie Viktor, Dr., W-5064 Rösrath (DE); Dompert, Wolfgang, Dr., W-5000 Köln 91 (DE); Sommermeyer, Henning, Dr., W-5000 Köln 80 (DE)

(56) Entgegenhaltungen:
- EP-A- 0 270 947
- EP-A- 0 352 613

## Beschreibung

Die Erfindung betrifft Azaheterocyclylmethyl-chromane, Verfahren zu ihrer Herstellung sowie ihre Verwendung in Arznemitteln, insbesondere als Mittel zur Bekämpfung von Erkrankungen des zentralen Nervensystems.

Es ist bereits bekannt, daß 2-Benzofuranylmethylderivate eine ZNS-Aktivität besitzen (vgl. DE 2 165 276).

EP 352 613 betrifft unter anderem N-substituierte 2-Aminomethyl-chroman-derivate, die eine hohe Affinität zu Serotonin-Rezeptoren des 5-HT₁-Typs aufweisen. Sie eignen sich daher zur Behandlung von Erkrankungen des zentralen Nervensystems, beispielsweise von Depressionen.

In EP 270 947 werden N-substituierte 2-Amino-tetralin-derivate beschrieben, die eine hohe Affinität zu Serotonin-Rezeptoren des 5-HT₁-Typs aufweisen und deshalb zur Behandlung von Erkrankungen des zentralen Nervensystems geeignet sind.

Außerdem ist in der Publikation Eur. J. Med. Chem. 22 (6), 539-544 die Verbindung 1-[(3,4-Dihydro-2H-1-benzopyran-2-yl)methyl]piperidin in Form ihres Hydrochlorides mit einer α-adrenerg blockierenden Wirkung beschrieben.

Die Erfindung betrifft Azaheterocyclylmethyl-chromane der allgemeinen Formel (I), 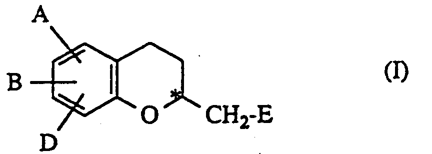
in welcher
- A, B und D: unabhängig voneinander
für Wasserstoff, Halogen, Cyano, Azido, Nitro, Difluormethyl, Trifluormethyl, Difluormethoxy, Trifluormethoxy, Hydroxy oder Carboxy stehen, oder
für geradkettiges oder verzweigtes Alkyl, Alkenyl, Acyl oder Alkoxycarbonyl mit jeweils bis zu 8 Kohlenstoffatomen stehen, oder
für eine Gruppe der Formel -NR¹R², -NR³-L-R⁴ oder -OR⁵ stehen,
worin
- R¹, R² und R³: gleich oder verschieden sind und Wasserstoff, geradkettiges oder verzweigtes Alkyl mit bis zu 8 Kohlenstoffatomen, Phenyl oder Benzyl bedeuten,
- L: die -CO- oder -SO₂-Gruppe bedeutet,
- R⁴: geradkettiges oder verzweigtes Alkyl mit bis zu 8 Kohlenstoffatomen oder Benzyl bedeutet, oder
Aryl mit 6 bis 10 Kohlenstoffatomen bedeutet, das gegebenenfalls durch Halogen, Hydroxy, Nitro, Cyano, Trifluormethyl, Trifluormethoxy oder durch geradkettiges oder verzweigtes Alkyl oder Alkoxy mit bis zu 6 Kohlenstoffatomen substituiert ist,
- R⁵: geradkettiges oder verzweigtes Alkyl oder Alkenyl mit jeweils bis zu 8 Kohlenstoffatomen bedeutet, die gegebenenfalls durch Cycloalkyl mit 3 bis 6 Kohlenstoffatomen oder Phenyl substituiert sind,
oder
- A: eine der oben aufgeführten Bedeutungen hat
und
- B und D: gemeinsam mit dem Aromaten einen 5- bis 7-gliedrigen gesättigten, partiell ungesättigten oder aromatischen Carbocyclus oder Heterocyclus mit bis zu 2 Heteroatomen aus der Reihe S, N oder O ausbilden, wobei diese gegebenenfalls bis zu 2 Carbonylfunktionen im Ring besitzen können und gegebenenfalls bis zu 2-fach gleich oder verschieden durch geradkettiges oder verzweigtes Alkyl, Alkenyl oder Alkoxy mit jeweils bis zu 6 Kohlenstoffatomen, Hydroxy, Cycloalkyl mit 3 bis 6 Kohlenstoffatomen, Phenyl, Halogen, Cyano, Nitro oder spiroartig durch einen Rest der Formel 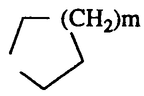 substituiert sind,
worin
- m: eine Zahl 1 oder 2 bedeutet,
- E: für einen heterocyclischen Rest der Formel 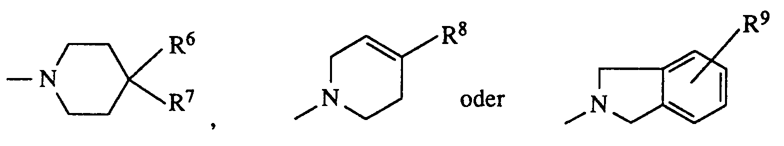 steht,
worin
- R⁶: Wasserstoff, Hydroxy, Halogen oder Phenyl bedeutet,
- R⁷ und R⁸: unabhängig voneinander
geradkettiges oder verzweigtes Alkyl mit bis zu 8 Kohlenstoffatomen bedeuten, das durch einen Rest der Formel 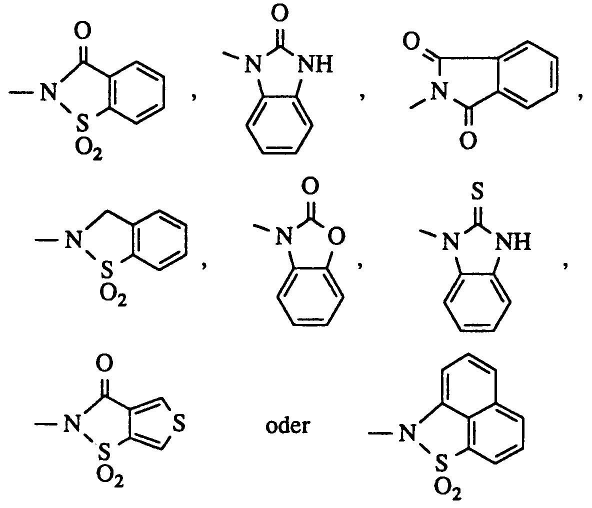 substituiert sein muß,

oder einen Rest der Formel 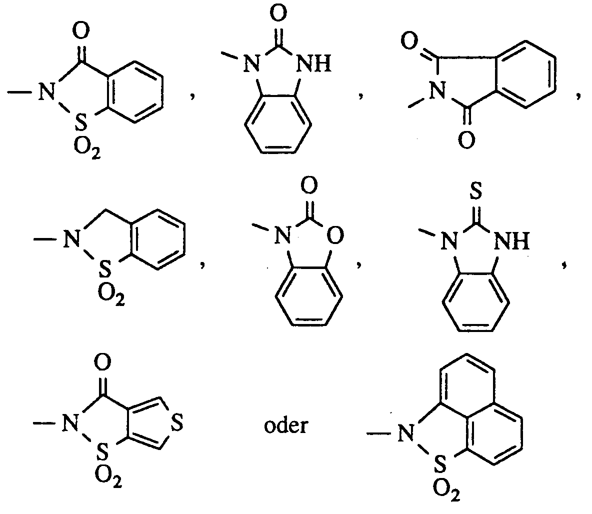 bedeuten
und
- R⁹: Wasserstoff, Halogen, Cyano, Nitro, Trifluormethyl, Trifluormethoxy, Hydroxy oder geradkettiges oder verzweigtes Alkyl oder Alkoxy mit jeweils bis zu 6 Kohlenstoffatomen bedeutet,
gegebenenfalls in einer isomeren Form,
und deren Salze.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Heterocyclus steht im allgemeinen für einen 5- bis 7-gliedrigen, bevorzugt 5- bis 6-gliedrigen, gesättigten oder ungesättigten Ring, der als Heteroatome bis zu 2 Sauerstoff- , Schwefel- und/oder Stickstoffatome enthalten kann. Bevorzugt sind 5- und 6-gliedrige Ringe mit einem Sauerstoff-, Schwefel- und/oder bis zu 2 Stickstoffatomen. Bevorzugt werden genannt: Thienyl, Furyl, Pyrrolyl, Pyrazolyl, Pyranyl, Pyridyl, Pyrimidyl, Pyrazinyl, Pyridazinyl, Thiazolyl, Oxazolyl, Imidazolyl, Isoxazolyl, Pyrrolidinyl, Piperidinyl, Piperazinyl, Morpholinyl oder Dioxanyl.

Im Rahmen der vorliegenden Erfindung können die erfindungsgemäßen Verbindungen in verschiedenen stereoisomeren Formen vorliegen. Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),
in welcher
- A, B und D: unabhängig voneinander
für Wasserstoff, Fluor, Chlor, Brom, Cyano, Trifluormethyl, Difluormethoxy, Trifluormethoxy oder Hydroxy stehen, oder
für geradkettiges oder verzweigtes Alkyl, Alkenyl, Acyl oder Alkoxycarbonyl mit jeweils bis zu 6 Kohlenstoffatomen stehen, oder
für eine Gruppe der Formel -NR¹R², -NR³-L-R⁴ oder -OR⁵ stehen,
worin
- R¹, R² und R³: gleich oder verschieden sind und Wasserstoff oder geradkettiges oder verzweigtes Alkyl mit bis zu 6 Kohlenstoffatomen bedeuten,
- L: die -CO- oder -SO₂-Gruppe bedeutet,
- R⁴: geradkettiges oder verzweigtes Alkyl mit bis zu 6 Kohlenstoffatomen oder Benzyl bedeutet, oder
Phenyl bedeutet, das gegebenenfalls durch Fluor, Chlor, Brom, Trifluormethyl, Trifluormethoxy, Hydroxy oder durch geradkettiges oder verzweigtes Alkyl oder Alkoxy mit jeweils bis zu 4 Kohlenstoffatomen substituiert ist,
- R⁵: geradkettiges oder verzweigtes Alkyl oder Alkenyl mit bis zu 6 Kohlenstoffatomen bedeutet, die gegebenenfalls durch Cyclopropyl, Cyclopentyl, Cyclohexyl oder Phenyl substituiert sind,
oder
- A: eine der oben aufgeführten Bedeutungen hat
und
- B und D: gemeinsam einen Rest der Formel 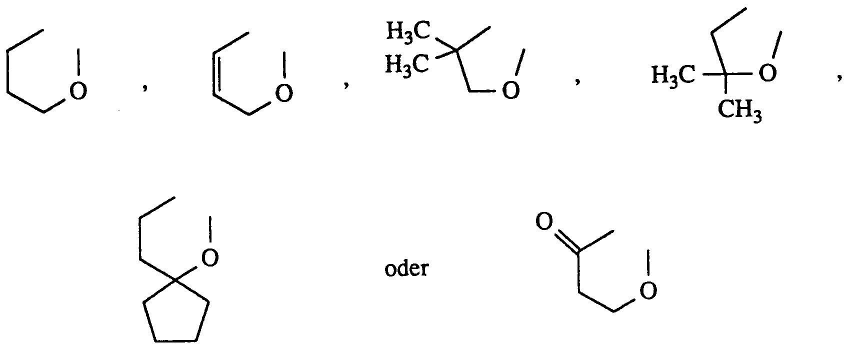 bilden,
- E: für einen heterocyclischen Rest der Formel 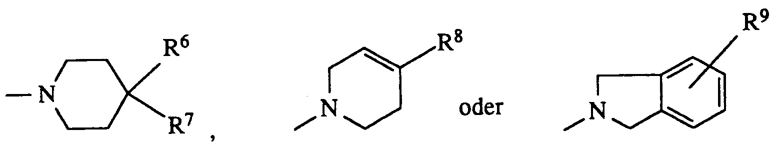 steht,
worin
- R⁶: Wasserstoff, Hydroxy, Fluor, Chlor, Brom oder Phenyl bedeutet,
- R⁷ und R⁸: unabhängig voneinander geradkettiges oder verzweigtes Alkyl mit bis zu 6 Kohlenstoffatomen bedeuten, das durch einen Rest der Formel 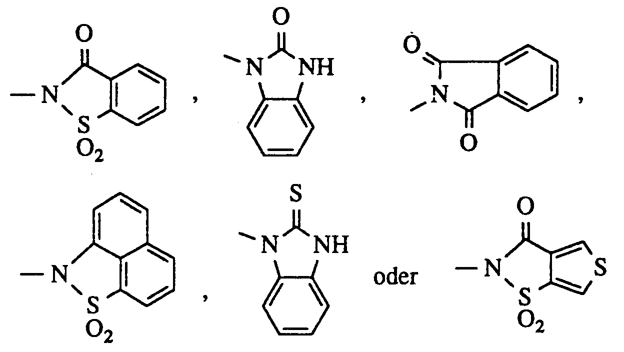 substituiert sein muß,
oder einen Rest der Formel 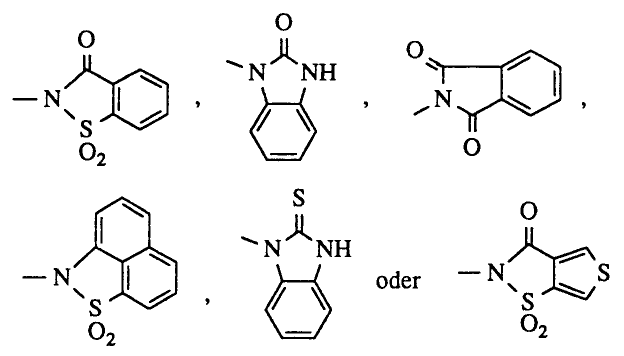 bedeuten,
und
- R⁹: Wasserstoff, Fluor, Chlor, Brom, Trifluormethyl, Hydroxy, geradkettiges oder verzweigtes Alkyl oder Alkoxy mit jeweils bis zu 4 Kohlenstoffatomen bedeutet,
gegebenenfalls in einer isomeren Form,
und deren Salze.

Besonders bevorzugt sind Verbindungen der allgemeinen Formel (I),
in welcher
- A, B und D: unabhängig voneinander
für Wasserstoff, Fluor, Chlor, Brom, Cyano, Trifluormethyl, Trifluormethoxy oder Hydroxy stehen, oder
für geradkettiges oder verzweigtes Alkyl oder Alkenyl mit jeweils bis zu 4 Kohlenstoffatomen stehen, oder
für eine Gruppe der Formel -NR¹R² oder -OR⁵stehen,
worin
- R¹ und R²: gleich oder verschieden sind und Wasserstoff oder geradkettiges oder verzweigtes Alkyl mit bis zu 4 Kohlenstoffatomen bedeuten,
- R⁵: geradkettiges oder verzweigtes Alkyl oder Alkenyl mit bis zu 4 Kohlenstoffatomen bedeutet, die gegebenenfalls durch Cyclopropyl oder Phenyl substitituiert sind,
oder
- A: die oben aufgeführten Bedeutungen hat
und
- B und D: gemeinsam einen Rest der Formel 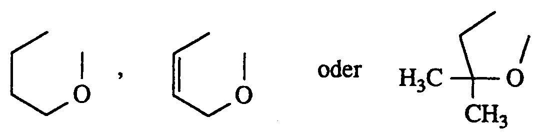 bilden,
- E: für einen heterocyclischen Rest der Formel 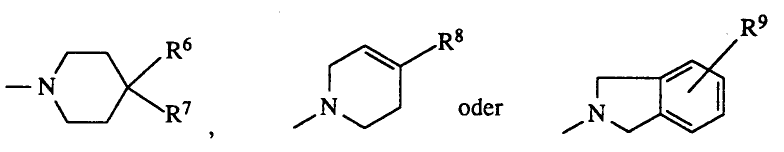 steht,
worin
- R⁶: Wasserstoff, Hydroxy, Fluor oder Chlor bedeutet,
- R⁷ und R⁸: unabhängig voneinander geradkettiges oder verzweigtes Alkyl mit bis zu 4 Kohlenstoffatomen bedeuten, das durch einen Rest der Formel 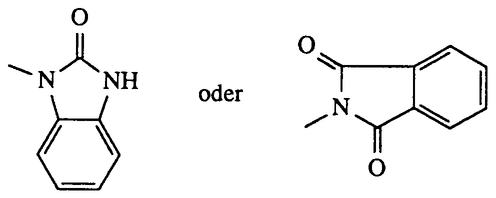 substituiert sein muß, 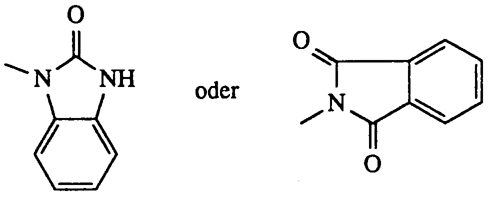
oder einen Rest der Formel bedeuten,
und
- R⁹: Wasserstoff, Fluor, Chlor, Trifluormethyl, Hydroxy, Methyl, Ethyl, Methoxy oder Ethoxy bedeutet,
gegebenenfalls in einer isomeren Form,
und deren Salze.

Außerdem wurden Verfahren zur Herstellung der erfindungsgemäßen Verbindungen der allgemeinen Formel (I) gefunden, dadurch gekennzeichnet, daß man
[A] Verbindungen der allgemeinen Formel (II) 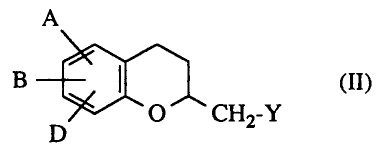 in welcher
   - A, B und D: die oben angegebene Bedeutung haben
   und
   - Y: für Hydroxy oder für eine typische Abgangsgruppe, wie beispielsweise Tosylat, Chlorid oder Mesylat, vorzugsweise für Tosylat steht,
   direkt mit Verbindungen der allgemeinen Formel (III)

   H-E (III),

   in welcher
   - E: die oben angegebene Bedeutung hat,
   in inerten Lösemitteln, in Anwesenheit einer Base und gegebenenfalls eines Hilfsstoffes (Katalysator, Starter) umsetzt,
   oder
[B] im Fall, daß R⁷ für einen Rest der Formel -T-R¹⁰ steht,
   worin
   - T: geradkettiges oder verzweigtes Alkyl mit bis zu 8 Kohlenstoffatomen bedeutet
   und
   - R¹⁰: für einen der unter R⁷ oben angegebenen heterocyclischen Reste steht,
   Verbindungen der allgemeinen Formel (IV), 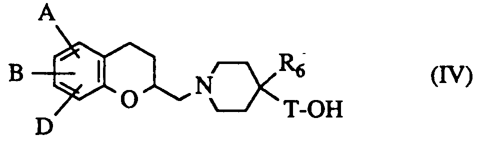 in welcher
   - A, B, D, R⁶ und T: die oben angegebene Bedeutung haben,
   mit Verbindungen der allgemeinen Formel (V)

   H-R¹⁰ (V)

   in welcher
   - R¹⁰: die oben angegebene Bedeutung hat,
   in inerten Losemitteln in einer Mitsunobu-Reaktion umsetzt,
   und gegebenenfalls Reduktionen nach üblichen Methoden durchführt,
   im Fall der Enantiomeren, entweder die Verbindungen der allgemeinen Formel (III) mit enantiomerenreinen Verbindungen der allgemeinen Formel (II) umsetzt, oder die entsprechenden Racemate der Verbindungen der allgemeinen Formel (I) nach den oben beschriebenen bekannten Methoden der Racematspaltung, beispielsweise durch Trennung über Salze mit enantiomerenreinen Säuren trennt
   und gegebenenfalls die Substituenten A, B und D ebenfalls nach bekannten Methoden derivatisiert.

Die erfindungsgemäßen Verfahren können durch folgendes Formelschema beispielhaft erläutert werden: 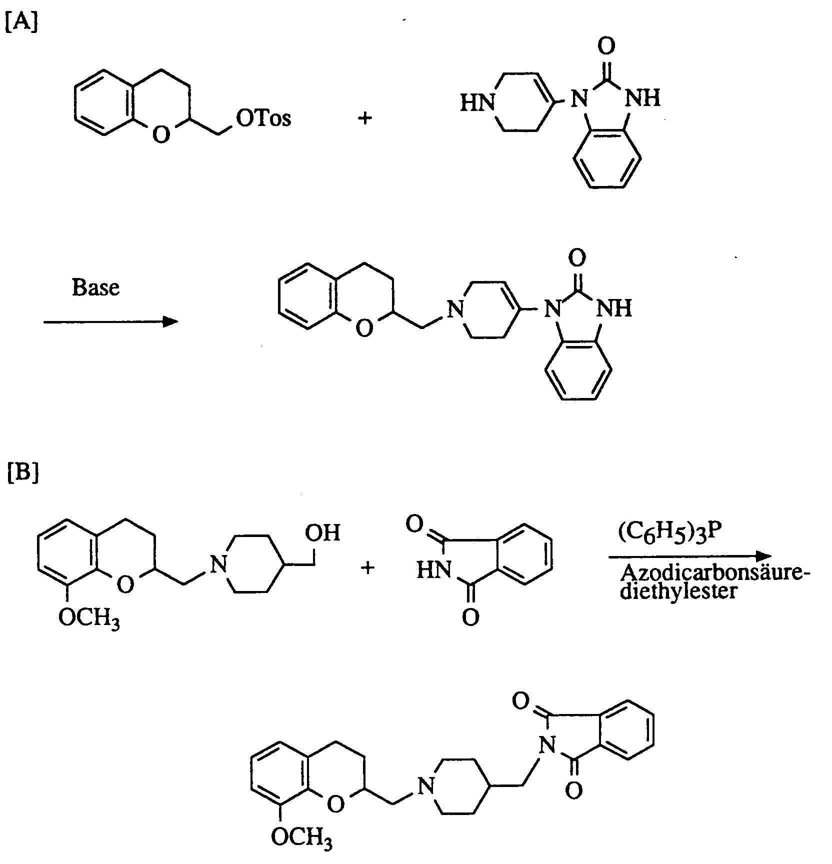
Als Lösemittel für die Umsetzung mit den Aminen der allgemeinen Formel (III) eignen sich die üblichen Lösemittel, die sich unter den Reaktionsbedingungen nicht verändern. Hierzu gehören bevorzugt Alkohle wie Methanol, Ethanol, Propanol oder Isopropanol, oder Ether wie Diethylether, Dioxan, Tetrahydrofuran, Glykoldimethylether oder Butylmethylether, oder Ketone wie Aceton oder Butanon, oder Amide wie Dimethylformamid oder Hexamethylphosphorsäuretriamid, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden. Bevorzugt sind Methanol, Ethanol, Propanol, Isopropanol, oder Dimethylformamid.

Als Basen eignen sich die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Triethylamin, Picolin, Pyridine oder N-Methylpiperidin, oder Amide wie Natriumamid oder Lithiumdiisopropylamid. Bevorzugt sind Natrium- und Kaliumcarbonat und Pyridin.

Die Base wird in einer Menge von 0,5 mol bis 10 mol, bevorzugt von 0,3 mol bis 3 mol bezogen auf 1 mol der Verbindungen der allgemeinen Formel (II) eingesetzt. Im Fall von Pyridin kann die Base auch als Lösemittel eingesetzt werden.

Die Reaktion wird im allgemeinen in einem Temperaturbereich von 0°C bis 150°C, bevorzugt von +20°C bis +110°C durchgeführt.

Die Umsetzung kann bei normalem, erhöhtem oder bei erniedrigtem Druck durchgeführt werden (z.B. 0,5 bis 5 bar). Im allgemeinen arbeitet man bei Normaldruck.

Die Reduktionen können im allgemeinen durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle, Platin, oder mit Hydriden oder Boranen in inerten Lösemitteln, gegebenenfalls in Anwesenheit eines Katalysators durchgeführt werden.

Bevorzugt wird die Reaktion mit Hydriden, wie komplexen Borhydriden oder Aluminiumhydriden durchgeführt. Besonders bevorzugt werden hierbei Natriumborhydrid, Lithiumaluminiumhydrid oder Natriumcyanoborhydrid eingesetzt.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden.

Als Katalysatoren bei der Reduktion mit Natriumcyanoborhydrid werden im allgemeinen Protonensäuren verwendet. Hierzu gehören bevorzugt anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit C₁-C₄-Alkylresten oder Arylresten wie beispielsweise Methansulfonsäure, Ethansulfonsäure, Benzolsulfonsäure oder Toluolsulfonsäure.

Die Mitsunobu-Reaktion verläuft im allgemeinen in einem der oben aufgeführten nicht protischen Lösemitteln, vorzugsweise Tetrahydrofuran, in Anwesenheit von Phosphanen, vorzugsweise Triphenylphosphan und Esterderivaten der Azodicarbonsäure, vorzugweise Azodicarbonsäurediethylester, in einem Temperaturbereich von 0°C bis +50°C, vorzugsweise bei Raumtemperatur und Normaldruck [vgl. hierzu O.Mitsunobu, Synthesis 1981,1].

Die Verbindungen der allgemeinen Formel (II) sind an sich bekannt oder können nach üblicher Methode hergestellt werden [vgl. DE 3 620 408 A, US 4 957 928, Farmaco, Ed. Sci. 42 (11), 805-813], wobei die enantiomerenreinen Verbindungen erhältlich sind, indem man bei der Herstellung die entsprechenden enantiomerenreinen Chroman-2-carbonsäuren und deren Derivate einsetzt [vgl. hierzu J. Labelled , Comp., Pharm. 24, 909, 1987].

Die Amine der allgemeinen Formel (III) sind bekannt, nach üblichen Methoden herstellbar oder käuflich [vgl. MSD Book 2, 2846 D; Beilstein 21 (2) 8].

Die Verbindungen der allgemeinen Formel (IV) sind größtenteils neu und können dann beispielsweise hergestellt werden, indem man die entsprechenden Verbindungen der allgemeinen Formel (VI) 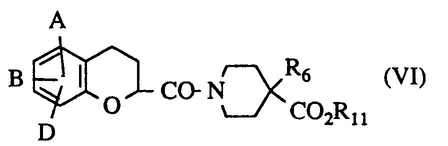
in welcher
- A, B, D und R⁶: die oben angegebene Bedeutung haben
und
- R¹¹: für C₁-C₄-Alkyl steht,
in inerten Lösemitteln reduziert.

Die Reduktion der Säureamide und Imide erfolgt mit Hydriden in inerten Lösemitteln oder mit Boranen, Diboranen oder ihren Komplexverbindungen.

Bevorzugt werden die Reaktionen mit Hydriden, wie komplexen Borhydriden oder Aluminiumhydriden sowie Boranen durchgeführt. Besonders bevorzugt werden hierbei Natriumborhydrid, Lithiumaluminiumhydrid, Natrium-bis-(2-methoxyethoxy)aluminiumhydrid oder Boran-Tetrahydrofuran eingesetzt.

Die Umsetzung kann bei normalem, erhöhtem oder bei erniedrigtem Druck durchgeführt werden (z.B. 0,5 bis 5 bar). Im allgemeinen arbeitet man bei Normaldruck.

Die Reduktion erfolgt im allgemeinen in einem Temperaturbereich von - 50°C bis zum jeweiligen Siedepunkt des Lösemittels, bevorzugt von -20°C bis +90°C.

Die Verbindungen der allgemeinen Formel (VI) sind teilweise bekannt oder neu und können beispielsweise hergestellt werden, indem man die entsprechenden aktivierten 2-Carbonsäurechromanderivate mit Piperidin-4-carbonsäureestern in inerten Lösemitteln, vorzugsweise Pyridin, in Anwesenheit einer der oben aufgeführten Basen, vorzugsweise Pyridin, umsetzt.

Die erfindungsgemäßen Verbindungen können als Wirkstoffe in Arzneimitteln verwendet werden. Die erfindungsgemäßen Stoffe haben eine besonders hohe Affinität zu cerebralen 5-Hydroxy-tryptamin-Rezeptoren vom 5-HT₁-Typ. Auch besitzen sie hohe Affinität an Dopamin-Rezeptoren vom D₂-Typ.

Die erfindungsgemäßen Stoffe zeigen überraschenderweise eine vorteilhafte Wirkung auf das Zentralnervensystem und können zur therapeutischen Behandlung von Menschen und Tieren verwendet werden.

Die in der vorliegenden Erfindung beschriebenen Verbindungen stellen somit Wirkstoffe zur Bekämpfung von Krankheiten dar, die durch Störungen des serotoninergen und dopaminergen Systems, insbesondere bei Involvierung von Rezeptoren, die hohe Affinität zu 5-Hydroxytryptamin (5-HT₁-Typ) und/oder zu Dopamin (D₂-Typ) besitzen, gekennzeichnet sind. Sie eignen sich daher zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuß- und Suchtmittelaufnahme. Weiterhin sind sie geeignet zur Beseitigung kognitiver Defizite, zur Verbesserung von Lern- und Gedächtnisleistungen und zur Behandlung der Alzheimer'schen Krankheit. Auch sind sie geeignet zur Bekämpfung von Psychosen (z.B- Schizophrenie, Marie). Gegenüber bekannten Neuroleptika besitzen sie ein geringeres Nebenwirkungspotential.

Weiterhin eignen sich diese Wirkstoffe auch zur Modulierung des cardiovaskulären Systems. Sie greifen auch in die Regulation der cerebralen Durchblutung ein und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien. Darüber hinaus können die Verbindungen zur Behandlung von akutem Schädel-Hirn Trauma verwendet werden. Ebenso können die erfindungsgemäßen Verbindungen zur Bekämfpung von Schmerzzuständen eingesetzt werden.

### Affinität zum 5-HT₁-Rezeptor

In Tabelle 1 wird beispielhaft die hohe Affinität der erfindungsgemäßen Verbindungen zu 5-Hydroxytryptamin-Rezeptoren vom Subtyp 1 dargestellt. Bei den angegebenen Werten handelt es sich um Daten, die aus Rezeptorbindungsstudien mit Kalbs-Hippocampus-Membranpräparationen ermittelt wurden. Als radioaktiv markierter Ligand wurde hierzu ³H-Serotonin verwendet.

**Tabelle [A]**

| Verbindung des Beispiels | Kᵢ (nmol/l) |
|---|---|
| 2 | 3 |
| 4 | 2 |

### Affinität zum 5-HT_{1A}-Rezeptor [W.U. Dompert et al., Naunyn-Schmiedeberg's Arch. Pharmacol. (1985), 328, 467-470].

Bei diesem Test wird die Bindung von ³H-Ipsapiron an 5-HT_{1A}-Rezeptoren in Kalbshippocampus-Membranen gemessen. Es wurde gefunden, daß die erfindungsgemäßen Verbindungen mit dem Radioliganden um die Bindung konkurrieren und diese hemmen.

**Tabelle [B]**

| Verbindung des Beispiels | Kᵢ (nmol/l) |
|---|---|
| 5 | 1,5 |
| 6 | 1,4 |

### Dopamin D₂-Rezeptortest

Dieser Test wird nach folgender Literaturstelle durchgeführt: Imafuku J. (1987), Brain Research 402; 331-338.

Dabei wird die Bindung des selektiven D₂-Rezeptor-Antagonisten ³H-Sulpirid an Membranen aus dem Striatum der Ratte gemessen. Verbindungen, die an Dopamin-D₂-Rezeptoren binden, hemmen konzentrationsabhängig die Bindung von ³H-Sulpirid. Aus den Verdrängungskurven werden IC₅₀-Werte ermittelt und daraus die Inhibitionskonstanten Kᵢ berechnet.

**Tabelle [C]**

| Verbindung des Beispiels | Kᵢ (nmol/l) |
|---|---|
| 2 | 1,8 |
| 3 | 0,4 |
| 6 | 2,3 |

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Die jeweils aufgeführten R_{f}- Werte wurden - sofern nicht anders vermerkt - durch Dünnschichtchromatographie auf Kieselgel (Alufolie, Kieselgel 60 F 254, Fa. E. Merck) ermittelt. Die Visualisierung der Substanzflecke geschah durch Betrachten unter UV-Licht und/oder durch Besprühen mit 1 %iger Kaliumpermanganat-Lösung.

Die Flash-Chromatographie wurde auf Kieselgel 60, 0,040 - 0,064 mm, Fa. E. Merck, durchgeführt (siehe Still et al., J. Org. Chem. 43, 2923, 1978; für einfachere Trennprobleme siehe Aldrichimica Acta 18, 25, 1985). Elution mit Lösemittelgradienten bedeutet: Beginnend mit der reinen, unpolaren Lösemittelgemischkomponente wird in einem steigendem Ausmaß die polare Laufmittelkomponente zugemischt, bis das gewünschte Produkt eluiert wird (DC-Kontrolle).

Bei allen Produkten wurde bei zuletzt ca. 0,1 Torr das Lösemittel abdestilliert. Salze wurden bei diesem Druck über Nacht über Kaliumhydroxid und/oder Phosphorpentoxid aufbewahrt.

### Ausgangsverbindungen

### Beispiel I

### 8-Methoxy-chroman-2-carbonsäure-(4-ethoxycarbonyl)-piperidid

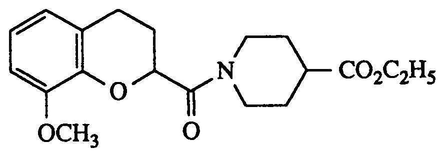

9,0 g (40 mmol) 8-Methoxy-chroman-2-carbonsäurechlorid wird in mehreren Portionen zu 6,3 g Piperidin-4-carbonsäureethylester (40 mmol) und 0,1 g 4-Dimethylaminopyridin in 20 ml wasserfreiem Pyridin zugegeben. Nach 40 Stunden bei Raumtemperatur wird auf Eis gegeben. Der nach 30 Minuten sich abscheidende Festkörper wird mit Wasser gewaschen und im Exsikkator getrocknet. Ausbeute: 6,2 g (45%)
Dieses Material wird ohne weitere Reinigung weiter eingesetzt.

### Beispiel II

### 2-Hydroxymethyl-8-methoxy-chroman

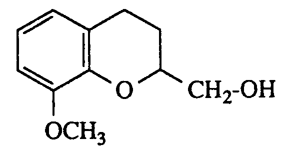

59,0 g (0,25 mol) 8-Methoxy-chroman-2-carbonsäureethylester werden in 525 ml wasserfreiem Tetrahydrofuran innerhalb 1 h unter Rühren bei 20°C zu der Suspension von 9,5 g (0,25 mol) Lithiumaluminiumhydrid in 525 ml wasserfreiem Diethylether getropft. Der Ansatz wird über Nacht gerührt und anschließend unter Kühlen nacheinander mit 9,5 ml Wasser, 9,5 ml 15%iger Natronlauge und 28,4 ml Wasser tropfenweise versetzt. Die organische Phase wird dekantiert und eingedampft. Der Rückstand wird 2 mal aus Dichiormethan / Petrolether umkristallisiert.
Ausbeute: 38,0 g (87%)
Schmp.: 57-58°C

### Beispiel III

### (2R)-2-Hydroxymethyl-chroman

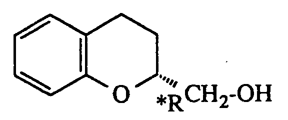

Zu der Lösung von 22,1 g (0,124 mol) (2R)-Chroman-2-carbonsäure (ee = 98,3%) in 210 ml wasserfreiem Tetrahydrofuran unter Argon werden innerhalb von 30 Minuten bei 0°C Innentemperatur 164 ml einer 1 M Boranlösung in Tetrahydrofuran zugetropft. Die Kühlung wird entfernt, und der Ansatz 4 h nachgerührt. Die Innentemperatur steigt währenddessen auf 34°C an. Anschließend werden 46 ml eines 1/1-Gemisches aus Tetrahydrofuran und Wasser unter Eiskühlung zugetropft. Nach Zugabe von 40,7 g wasserfreiem Kaliumcarbonat und kräftigem Rühren wird die Tetrahydrofuranlösung dekantiert und im Wasserstrahlvakuum eingeengt. Kurzwegdestillation liefert 18,8 g farbloses 2R-Hydroxymethylchroman vom Sdp. 77-78°C/0,15 mbar.
ee > 99%.

### Beispiel IV

### (2S)-2-Hydroxymethyl-chroman

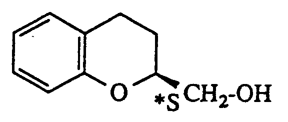

In Analogie zur Vorschrift des Beispiels III wird die Titelverbindung aus (2S)-2-Chroman-2-carbonsäure hergestellt.
ee >99%
Kp.: 79-81°C/0,15 mbar

### Beispiel V

### (2R)-2-Tosyloxymethyl-chroman

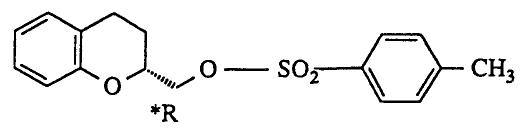

Zu 12,8 g (0,078 mol) (2R)-2-Hydroxymethylchroman (Beispiel II) in 50 ml wasserfreiem Pyridin werden unter Rühren und Eiskühlung 15,63 g (0,082 mol) 4-Toluolsulfochlorid portionsweise gegeben. Nach Stehenlassen über Nacht wird der Ansatz in Eiswasser eingetragen und mit Diethylether extrahiert Die Etherphase wird 2 mal mit 5%iger eiskalter Salzsäure und anschließend mit gesättigter Kochsalzlösung gewaschen, über wasserfreiem Natriumsulfat getrocknet und im Wasserstrahlvakuum eingedampft 22,4 g einheitlicher 4-Toluolsulfonsäureester des 2R-2-Hydroxymethylchromans werden erhalten.
R_{f} = 0,6 (Toluol/Essigester 3:1) Öl
Schmp.: 62-65°C (PE/Dichlormethan) [α]_{D} = -51,1° (c=1, Chloroform)

### Beispiel VI

### (2S)-2-Tosyloxymethyl-chroman

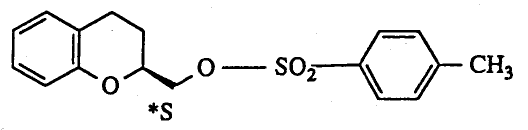

In Analogie zur Vorschrift des Beispiels V wird die Titelverbindung aus Beispiel IV hergestellt.
R_{f} = 0,6 (Toluol/Essigester 3:1) Öl

### Beispiel VII

### 8-Methoxy-2-tosyloxymethyl-chroman

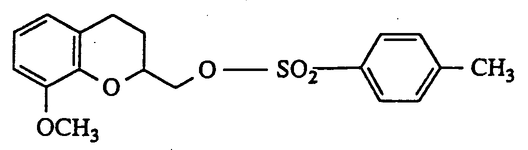

Schmp.: 115-117°C (aus Dichlormethan)

### Beispiel VIII:

### 2-Phthalimidomethyl-8-methoxy-chroman

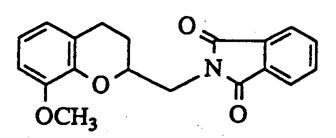

Durch Umsetzung der Verbindung aus Beispiel II mit Phthalimid in Gegenwart äquimolarer Mengen Triphenylphosphan und Azodicarbonsäurediethylester in Tetrahydrofuran wird das gewünschte Produkt in 80 % Ausbeute in Form eines Sirups erhalten, der direkt weiter umgesetzt wird.

R_{f} = 0,46 (Toluol/Essigester 3:1)

### Beispiel IX

### 2-[(4-Hydroxymethyl)-piperidin-1-yl-methyl]-8-methoxy-chroman Hydrochlorid

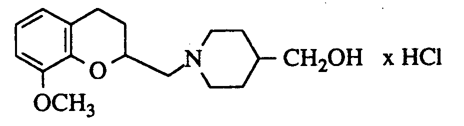

5,2 g (15 mmol) der Verbindung aus Beispiel I in 31 ml Toluol werden mit 31 ml einer 3,4 M Lösung von Natrium-bis-(2-methoxyethoxy)-dihydroaluminat in Toluol versetzt und 18 h bei 50°C unter Argon gerührt. Nach Verdünnen mit Toluol wird mit 10 ml eines 1:1 Gemisches aus Tetrahydrofuran und Wasser hydrolysiert. Filtration und Flashchromatographie des Filtrates (Kieselgel, Toluol/i-Propanol-Gradient 100:0 bis 50:50) liefert das Produkt als freie Base, 2,8 g (64%) in Form eines Sirups.
Durch Behandeln mit etherischer Salzsäure wird das Hydrochlorid erhalten, das aus Acetonitril umkristallisiert wird.
Schmp.: 107 - 112°C (nach Unkrist. aus Acetonitril)
IR (KBr): 3510, 3301(b), 2945, 2548(b), 1630(w), 1583(w), 1481
In Analogie zu den Vorschriften der Beispiele I und IX werden die in Tabelle I aufgeführten Verbindungen hergestellt:

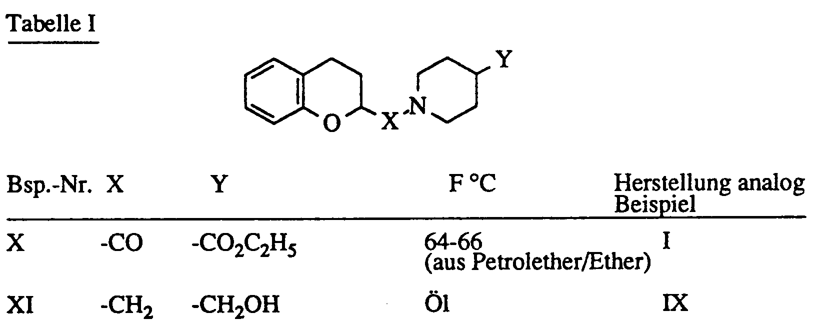

### Herstellungsbeispiele

### Beispiel 1

### 2-(1H-2,3-dihydro-2-indol-2-yl-methyl)-8-methoxychroman Hydrochlorid

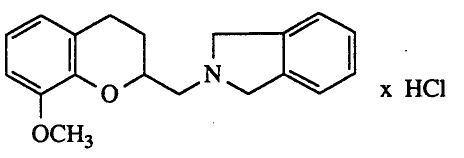

Zu 2,5 g (68 mmol) Lithiumaluminumhydrid in 80 ml Diethylether werden 7,3 g (23 mmol) der Verbindung aus Beispiel VIII in 50 ml Tetrahydrofuran zugetropft. Die Mischung wird 5 Stunden zum Rückfluß erhitzt und anschließend 15 Stunden bei Raumtemperatur stehen gelassen. Es werden 10 ml Wasser in 30 ml Tetrahydrofuran, gefolgt von 5 ml 45% Natronlauge zugetropft. Nach Filtration über Kieselgur und Nachwaschen des Feststoffs mit Toluol/Essigester 1/1 wird ein Filtrat erhalten, das am Rotationsverdampfer eingeengt wird. Flashchromatographie (Kieselgel, Toluol/Essigester-Gradient 100:0 bis 75:25) liefert 6,2 g (93%) des gewünschten Produkts als freie Base (Sirup).
Hieraus wird das Hydrochlorid durch Behandeln mit etherischer Salzsäure gewonnen.
Schmp.: 256-258°C (nach Umkrist. aus 2-Propanol)
R_{f} = 0,35 (Kieselgel, Toluol/Essigester 1:1)
MS (EI): 295, 132 (100%), 105, 36

### Beispiel 2

### 2-[4-(Isoindol-1,3-dion-2-yl)methyl-piperidin-1-yl-methyl)-8-methoxychroman Oxalat Hydrat

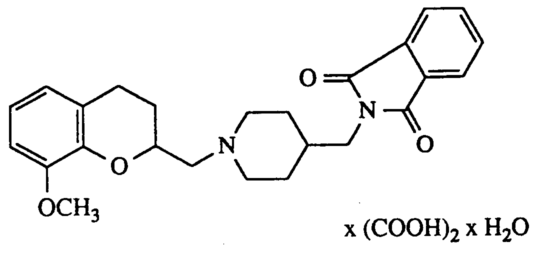

4,1 g (14 mmol) der Verbindung aus Beispiel IX, 4,1 g (15 mmol) Triphenylphosphan und 2,3 g (15 mmol) Phthalimid werden in 6 ml trocknem Tetrahydrofuran gelöst. Hierzu werden bei Raumtemperatur 2,7 g Azodicarbonsäurediethylester in 10 ml Tetrahydrofuran zugetropft. Nach 10 Tagen bei Raumtemperatur wird eingeengt und der Rückstand mit Cyclohexan digeriert. Ungelöstes wird abfiltriert und das Filtrat am Rotationsverdampfer eingeengt. Chromatographie (Kieselgel, Toluol/Essigester 100:0 bis 50:50) und Rechromatographie (Kieselgel, Dichlormethan/2-Propanol 50:1 bis 10:1) liefert das gewünschte Produkt in Form eines Öls (1,15 g). Hieraus wird durch Behandeln mit etherischer Salzsäure das Hydrochlorid gefällt (1,15 g, 16%). Zur weiteren Reinigung wird die Base hieraus mit Natriumhydrogencarbonatlösung freigesetzt. Das durch Versetzen mit Oxalsäure-Dihydrat in ethanolischer Lösung zugängliche Oxalat wird aus Acetonitril umkristallisiert. Nochmalige Umkristallisation aus 2-Propanol liefert nach Abkühlen auf -36°C 0,30 g analysenreine Titelverbindung.
Schmp.: >70°C (Zers.)
R_{f} = 0,3 (Kieselgel, Dichlormethan/2-Propanol 20:1)

### Beispiel 3

### 1-(Chroman-2-yl-methyl)-4-(2-oxo-1-benzimidazolyl)-piperidin

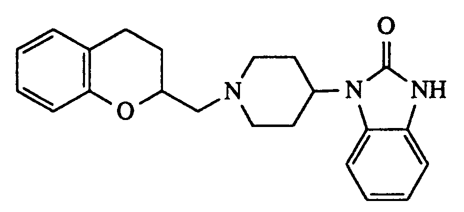

Das Gemisch aus 8,8 g (27,7 mmol) (Chroman-2R,S-yl-methyl)-4-toluolsulfonsäureester, 2,1 g (20 mmol) wasserfreiem Natriumcarbonat und 6,5 g (30 mmol) 4-(2-Oxo-1-benzimidazolinyl)-piperidin in 70 ml wasserfreiem Dimethylformamid wird 6 h bei 110°C gerührt und anschließend auf Eis (250 g) gegossen. Nach Extrahieren mit Essigsäureethylester, Waschen der organischen Extrakte mit Wasser, Trocknen über wasserfreiem Natriumsulfat und Eindampfen der organischen Phase im Wasserstrahlvakuum werden 9,8 g fast einheitliches kristallines Rohprodukt erhalten, das zur Analyse 2 mal aus Dichlormethan / Petrolether umkristallisiert wird.
Schmp.: 107-109°C (Kap.)

In Analogie zu der Vorschrift des Beispiels 3 wurden die in den Tabellen 1 und 2 aufgeführten Beispiele hergestellt.

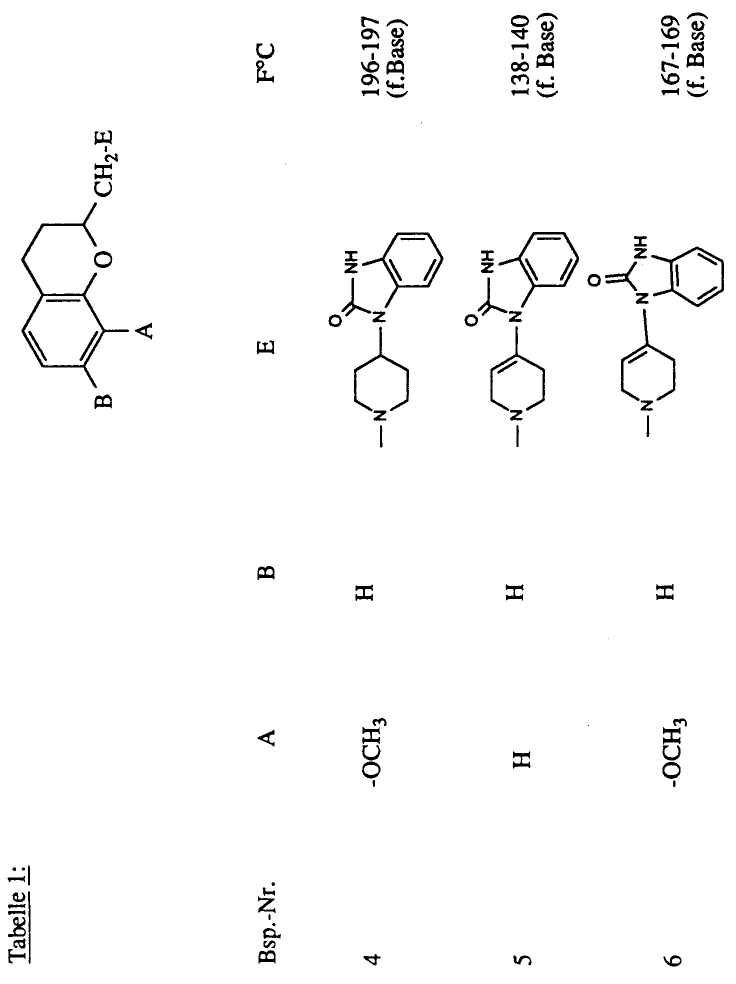

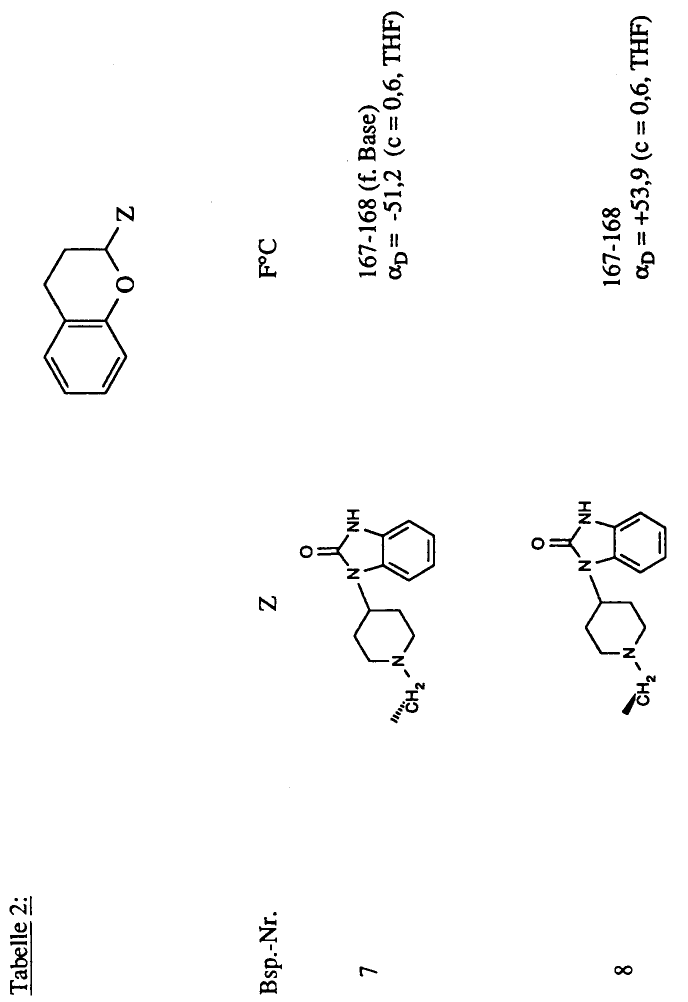

## Patentansprüche

1. Azaheterocyclylmethyl-chromane der allgemeinen Formel 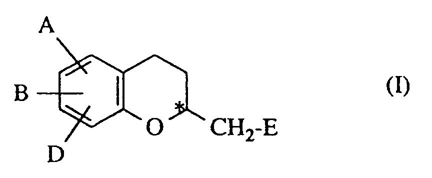 in welcher
A, B und D unabhängig voneinander
für Wasserstoff, Halogen, Cyano, Azido, Nitro, Difluormethyl, Trifluormethyl, Difluormethoxy, Trifluormethoxy, Hydroxy oder Carboxy stehen, oder
für geradkettiges oder verzweigtes Alkyl, Alkenyl, Acyl oder Alkoxycarbonyl mit jeweils bis zu 8 Kohlenstoffatomen stehen, oder
für eine Gruppe der Formel -NR¹R², -NR³-L-R⁴ oder -OR⁵ stehen,
worin
R¹, R² und R³ gleich oder verschieden sind und Wasserstoff, geradkettiges oder verzweigtes Alkyl mit bis zu 8 Kohlenstoffatomen, Phenyl oder Benzyl bedeuten,
L die -CO- oder -SO₂-Gruppe bedeutet,
R⁴ geradkettiges oder verzweigtes Alkyl mit bis zu 8 Kohlenstoffatomen oder Benzyl bedeutet, oder Aryl mit 6 bis 10 Kohlenstoffatomen bedeutet, das gegebenenfalls durch Halogen, Hydroxy, Nitro, Cyano, Trifluormethyl, Trifluormethoxy oder durch geradkettiges oder verzweigtes Alkyl oder Alkoxy mit bis zu 6 Kohlenstoffatomen substituiert ist,
R⁵ geradkettiges oder verzweigtes Alkyl oder Alkenyl mit jeweils bis zu 8 Kohlenstoffatomen bedeutet, die gegebenenfalls durch Cycloalkyl mit 3 bis 6 Kohlenstoffatomen oder Phenyl substituiert sind,
oder
A eine der oben aufgeführten Bedeutungen hat
und
B und D gemeinsam mit dem Aromaten einen 5- bis 7-gliedrigen gesättigten, partiell ungesättigten oder aromatischen Carbocyclus oder Heterocyclus mit bis zu 2 Heteroatomen aus der Reihe S, N oder O ausbilden, wobei diese gegebenenfalls bis zu 2 Carbonylfunktionen im Ring besitzen können und gegebenenfalls bis zu 2-fach gleich oder verschieden durch geradkettiges oder verzweigtes Alkyl, Alkenyl oder Alkoxy mit jeweils bis zu 6 Kohlenstoffatomen, Hydroxy, Cycloalkyl mit 3 bis 6 Kohlenstoffatomen, Phenyl, Halogen, Cyano, Nitro oder spiroartig durch einen Rest der Formel 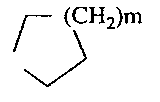 substituiert sind,
worin
m eine Zahl 1 oder 2 bedeutet,
E für einen heterocyclischen Rest der Formel 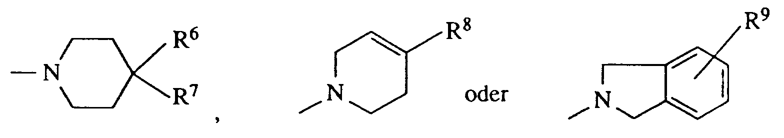 steht,
worin
R⁶ Wasserstoff, Hydroxy, Halogen oder Phenyl bedeutet,
R⁷ und R⁸ unabhängig voneinander
geradkettiges oder verzweigtes Alkyl mit bis zu 8 Kohlenstoffatomen bedeuten, das durch einen Rest der Formel 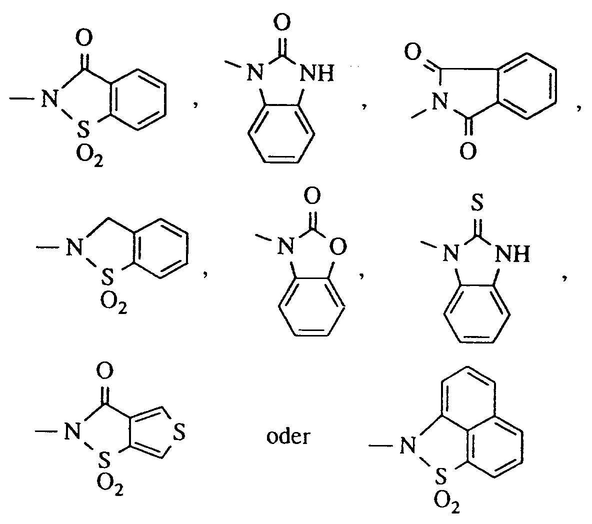 substituiert sein muß,
oder einen Rest der Formel 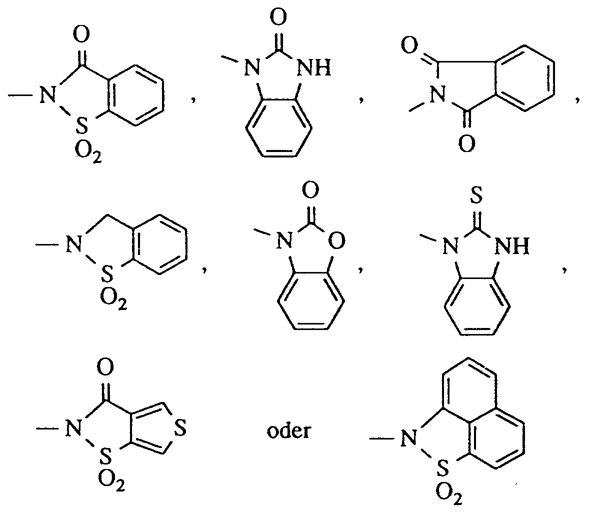 bedeuten
und
R⁹ Wasserstoff, Halogen, Cyano, Nitro, Trifluormethyl, Trifluormethoxy, Hydroxy oder geradkettiges oder verzweigtes Alkyl oder Alkoxy mit jeweils bis zu 6 Kohlenstoffatomen bedeutet,
gegebenenfalls in einer isomeren Form,
und deren Salze.

2. Azaheterocyclylmethyl-chromane nach Anspruch 1, wobei
A, B und D unabhängig voneinander für Wasserstoff, Fluor, Chlor, Brom, Cyano, Trifluormethyl, Difluormethoxy, Trifluormethoxy oder Hydroxy stehen, oder
für geradkettiges oder verzweigtes Alkyl, Alkenyl, Acyl oder Alkoxycarbonyl mit jeweils bis zu 6 Kohlenstoffatomen stehen, oder für eine Gruppe der Formel -NR¹R², -NR³-L-R⁴ oder -OR⁵ stehen,
worin
R¹, R² und R³ gleich oder verschieden sind und Wasserstoff oder geradkettiges oder verzweigtes Alkyl mit bis zu 6 Kohlenstoffatomen bedeuten,
L die -CO- oder -SO₂-Gruppe bedeutet,
R⁴ geradkettiges oder verzweigtes Alkyl mit bis zu 6 Kohlenstoffatomen oder Benzyl bedeutet, oder Phenyl bedeutet, das gegebenenfalls durch Fluor, Chlor, Brom, Trifluormethyl, Trifluormethoxy, Hydroxy oder durch geradkettiges oder verzweigtes Alkyl oder Alkoxy mit jeweils bis zu 4 Kohlenstoffatomen substituiert ist,
R⁵ geradkettiges oder verzweigtes Alkyl oder Alkenyl mit bis zu 6 Kohlenstoffatomen bedeutet, die gegebenenfalls durch Cyclopropyl, Cyclopentyl, Cyclohexyl oder Phenyl substituiert sind,
oder
A eine der oben aufgeführten Bedeutungen hat
und
B und D gemeinsam einen Rest der Formel 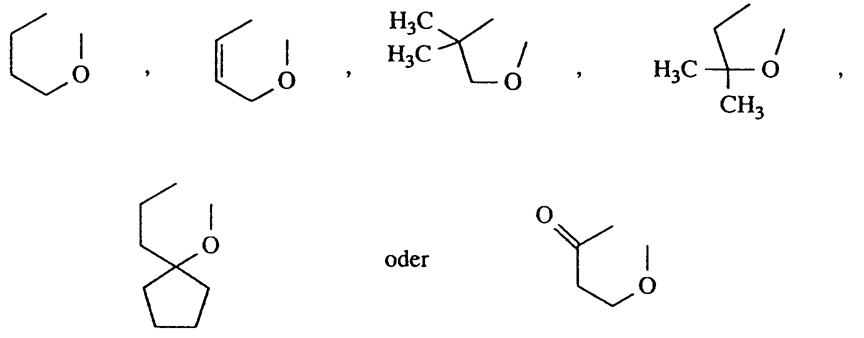 bilden,
E für einen heterocyclischen Rest der Formel 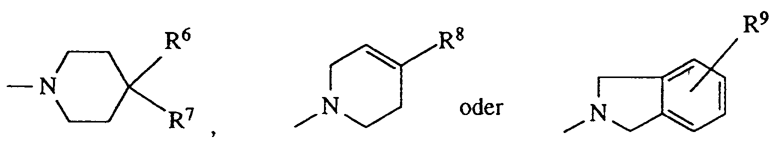 steht,
worin
R⁶ Wasserstoff, Hydroxy, Fluor, Chlor, Brom oder Phenyl bedeutet,
R⁷ und R⁸ unabhängig voneinander geradkettiges oder verzweigtes Alkyl mit bis zu 6 Kohlenstoffatomen bedeuten, das durch einen Rest der Formel 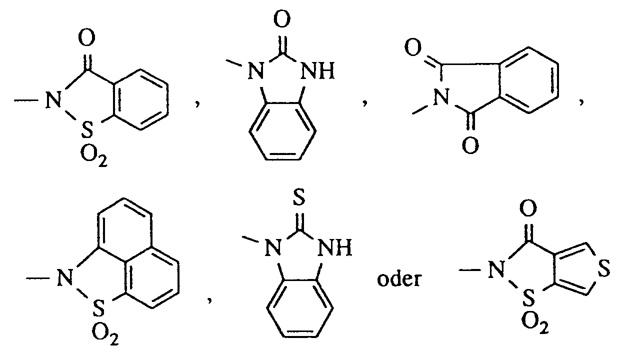 substituiert sein muß,
oder einen Rest der Formel 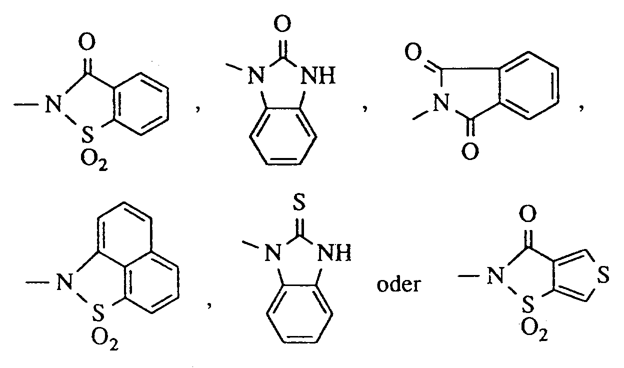 bedeuten,
und
R⁹ Wasserstoff, Fluor, Chlor, Brom, Trifluormethyl, Hydroxy, geradkettiges oder verzweigtes Alkyl oder Alkoxy mit jeweils bis zu 4 Kohlenstoffatomen bedeutet,
gegebenenfalls in einer isomeren Form,
und deren Salze.

3. Azaheterocyclylmethyl-chromane nach Anspruch 1, wobei
A, B und D unabhängig voneinander
für Wasserstoff, Fluor, Chlor, Brom, Cyano, Trifluormethyl, Trifluormethoxy oder Hydroxy stehen, oder
für geradkettiges oder verzweigtes Alkyl oder Alkenyl mit jeweils bis zu 4 Kohlenstoffatomen stehen, oder
für eine Gruppe der Formel -NR¹R² oder -OR⁵stehen,
worin
R¹ und R² gleich oder verschieden sind und Wasserstoff oder geradkettiges oder verzweigtes Alkyl mit bis zu 4 Kohlenstoffatomen bedeuten,
R⁵ geradkettiges oder verzweigtes Alkyl oder Alkenyl mit bis zu 4 Kohlenstoffatomen bedeutet, die gegebenenfalls durch Cyclopropyl oder Phenyl substitituiert sind,
oder
A die oben aufgeführten Bedeutungen hat
und
B und D gemeinsam einen Rest der Formel 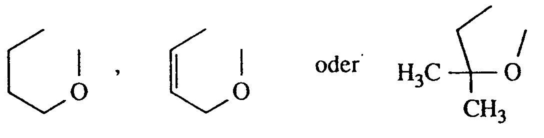 bilden,
E für einen heterocyclischen Rest der Formel 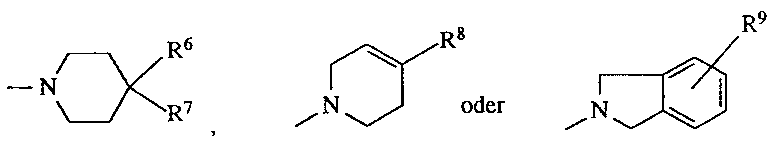 steht,
worin
R⁶ Wasserstoff, Hydroxy, Fluor oder Chlor bedeutet,
R⁷ und R⁸ unabhängig voneinander geradkettiges oder verzweigtes Alkyl mit bis zu 4 Kohlenstoffatomen bedeuten, das durch einen Rest der Formel 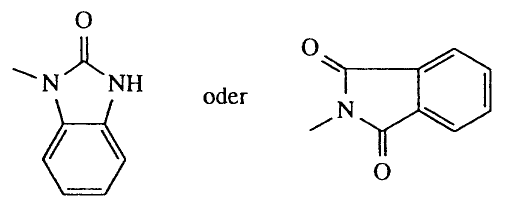 substituiert sein muß,
oder einen Rest der Formel 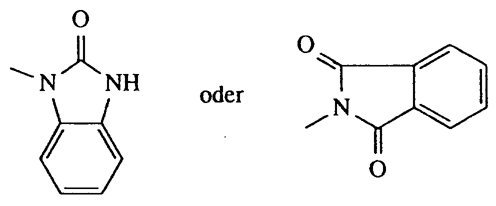 bedeuten,
und
R⁹ Wasserstoff, Fluor, Chlor, Trifluormethyl, Hydroxy, Methyl, Ethyl, Methoxy oder Ethoxy bedeutet,
gegebenenfalls in einer isomeren Form,
und deren Salze.

4. Azaheterocyclylmethyl-chromane nach Anspruch 1-3 zur therapeutischen Anwendung.

5. Verfahren zur Herstellung von Azaheterocyclylmethyl-chromanen nach Anspruch 1 - 3, dadurch gekennzeichnet, daß man
[A] Verbindungen der allgemeinen Formel (II) 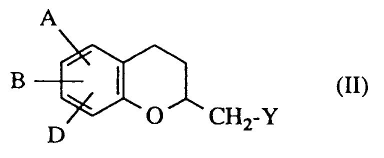 in welcher
A, B und D die in Anspruch 1 angegebene Bedeutung haben
und
Y für Hydroxy oder
für eine typische Abgangsgruppe, wie beispielsweise Tosylat, Chlorid oder Mesylat, vorzugsweise für Tosylat steht,
direkt mit Verbindungen der allgemeinen Formel (III)
H-E (III),
in welcher
E die in Anspruch 1 angegebene Bedeutung hat,
in inerten Lösemitteln, in Anwesenheit einer Base und gegebenenfalls eines Hilfsstoffes (Katalysator, Starter) umsetzt,
oder
[B] im Fall, daß R⁷ für einen Rest der Formel -T-R¹⁰ steht,
worin
T geradkettiges oder verzweigtes Alkyl mit bis zu 8 Kohlenstoffatomen bedeutet
und
R¹⁰ für einen der unter R⁷ oben angegebenen heterocyclischen Reste steht,
Verbindungen der allgemeinen Formel (IV), 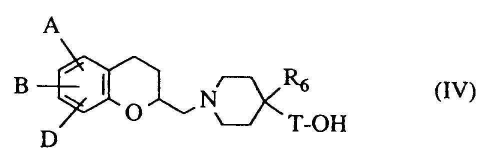 in welcher
A, B, D, R⁶ und T die oben angegebene Bedeutung haben,
mit Verbindungen der allgemeinen Formel (V)
H-R¹⁰ (V)
in welcher
R¹⁰ die oben angegebene Bedeutung hat,
in inerten Lösemitteln in einer Mitsunobu-Reaktion umsetzt,
und gegebenenfalls Reduktionen nach üblichen Methoden durchführt,
im Fall der Enantiomeren, entweder die Verbindungen der allgemeinen Formel (III) mit enantiomerenreinen Verbindungen der allgemeinen Formel (II) umsetzt, oder die entsprechenden Racemate der Verbindungen der allgemeinen Formel (I) nach bekannten Methoden der Racematspaltung, beispielsweise durch Trennung über Salze mit enantiomerenreinen Säuren trennt
und gegebenenfalls die Substituenten A, B und D ebenfalls nach bekannten Methoden derivatisiert.

6. Arzneimittel enthaltend mindestens ein Azaheterocyclylmethyl-chroman nach Anspruch 1 - 3.

7. Arzneimittel nach Anspruch 6 zur Bekämpfung von Krankheiten, die durch eine Störung des serotoninergen Systems gekennzeichnet sind.

8. Arzneimittel nach Anspruch 6 zur Bekämpfung von Krankheiten, die durch eine Störung des dopaminergen Systems gekennzeichnet sind.

9. Verfahren zur Herstellung von Arzneimitteln nach Anspruch 6, dadurch gekennzeichnet, daß man die Azaheterocyclylmethyl-chromane gegebenenfalls mit Hilfe von üblichen Hilfs- und Trägerstoffen in eine geeignete Applikationsform überführt.

10. Verwendung der Azaheterocyclyl-chromane nach Anspruch 1 - 3 zur Herstellung von Arzneimitteln.

## Claims

1. Azaheterocyclylmethyl-chromans of the general formula 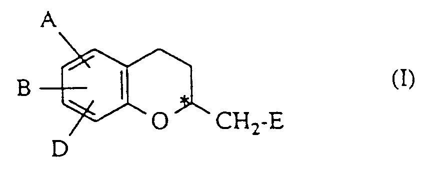 in which
A, B and D independently of one another
represent hydrogen, halogen, cyano, azido, nitro, difluoromethyl, trifluoromethyl, difluoromethoxy, trifluoromethoxy, hydroxyl or carboxyl, or
represent straight-chain or branched alkyl, alkenyl, acyl or alkoxycarbonyl having, in each case, up to 8 carbon atoms, or
represent a group of the formula -NR¹R², -NR³-L-R⁴ or -OR⁵,
wherein
R¹, R² and R³ are identical or different and denote hydrogen, straight-chain or branched alkyl having up to 8 carbon atoms, phenyl or benzyl,
L denotes the -CO- or -SO₂- group,
R⁴ denotes straight-chain or branched alkyl having up to 8 carbon atoms or benzyl, or denotes aryl having 6 to 10 carbon atoms, which is optionally substituted by halogen, hydroxyl, nitro, cyano, trifluoromethyl or trifluoromethoxy or by straight-chain or branched alkyl or alkoxy having up to 6 carbon atoms, and
R⁵ denotes straight-chain or branched alkyl or alkenyl having, in each case, up to 8 carbon atoms, which are optionally substituted by cycloalkyl having 3 to 6 carbon atoms or phenyl,
or
A has one of the abovementioned meanings
and
B and D together with the aromatic radical form a 5-membered to 7-membered saturated, partially unsaturated or aromatic carbocycle or heterocycle having up to 2 hetero atoms from the series S, N or O, the said cyclic radicals optionally being able to have up to 2 carbonyl functions in the ring and optionally being substituted, by up to 2 identical or different substituents, by straight-chain or branched alkyl, alkenyl or alkoxy having, in each case, up to 6 carbon atoms, hydroxyl, cycloalkyl having 3 to 6 carbon atoms, phenyl, halogen, cyano or nitro or, in a spiro-like manner, by a radical of the formula 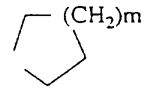 wherein
m denotes a number 1 or 2, and
E represents a heterocyclic radical of the formula 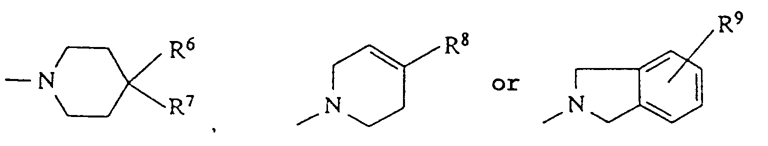 wherein
R⁶ denotes hydrogen, hydroxyl, halogen or phenyl,
R⁷ and R⁸ independently of one another denote straight-chain or branched alkyl having up to 8 carbon atoms, which must be substituted by a radical of the formula 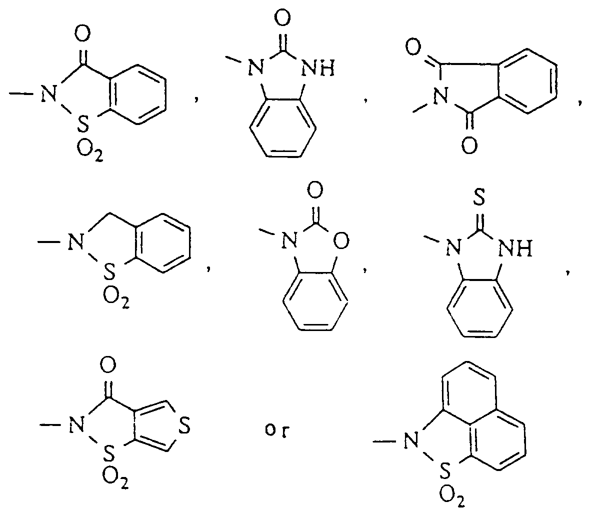 or denote a radical of the formula 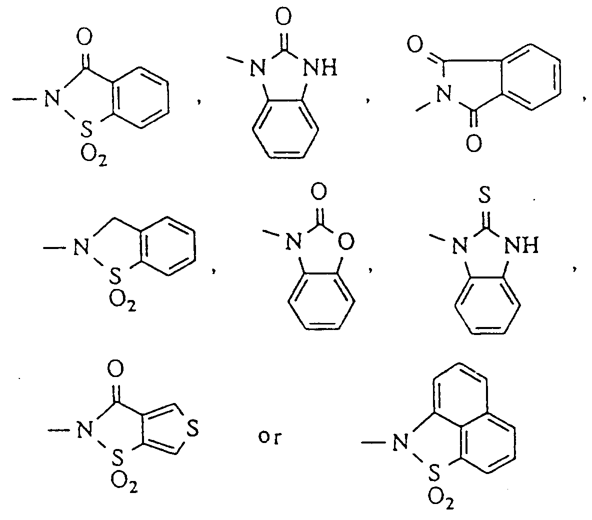 and
R⁹ denotes hydrogen, halogen, cyano, nitro, trifluoromethyl, trifluoromethoxy, hydroxyl or straight-chain or branched alkyl or alkoxy having, in each case, up to 6 carbon atoms,
optionally in an isomeric form,
and their salts.

2. Azaheterocyclylmethyl-chromans according to Claim 1, wherein
A, B and D independently of one another
represent hydrogen, fluorine, chlorine, bromine, cyano, trifluoromethyl, difluoromethoxy, trifluoromethoxy or hydroxyl, or
represent straight-chain or branched alkyl, alkenyl, acyl or alkoxycarbonyl having, in each case, up to 6 carbon atoms, or
represent a group of the formula -NR¹R², -NR³⁻ -L-R⁴ or -OR⁵,
wherein
R¹, R² and R³ are identical or different and denote hydrogen or straight-chain or branched alkyl having up to 6 carbon atoms,
L denotes the -CO- or -SO₂- group,
R⁴ denotes straight-chain or branched alkyl having up to 6 carbon atoms or benzyl, or denotes phenyl which is optionally substituted by fluorine, chlorine, bromine, trifluoromethyl, trifluoromethoxy or hydroxyl or by straight-chain or branched alkyl or alkoxy having, in each case, up to 4 carbon atoms, and
R⁵ denotes straight-chain or branched alkyl or alkenyl having up to 6 carbon atoms, which are optionally substituted by cyclopropyl, cyclopentyl, cyclohexyl or phenyl,
or
A has one of the abovementioned meanings
and
B and D together form a radical of the formula 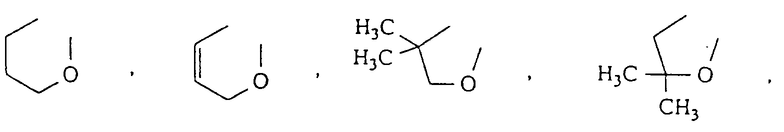 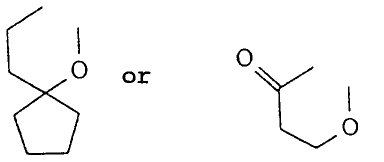 and
E represents a heterocyclic radical of the formula 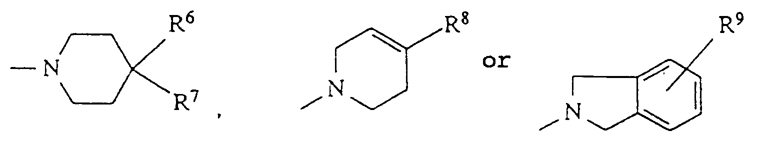 wherein
R⁶ denotes hydrogen, hydroxyl, fluorine, chlorine, bromine or phenyl,
R⁷ and R⁸ independently of one another denote straight-chain or branched alkyl having up to 6 carbon atoms, which must be substituted by a radical of the formula 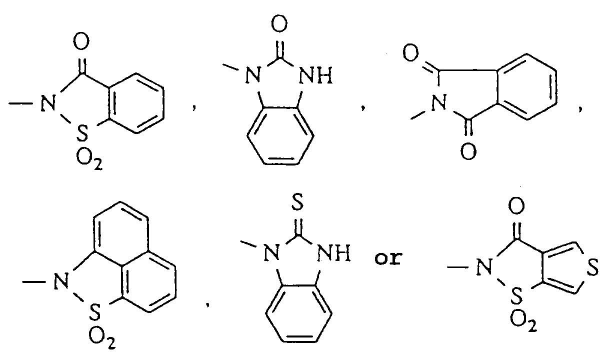 or denote a radical of the formula 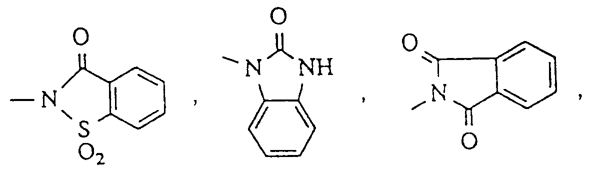 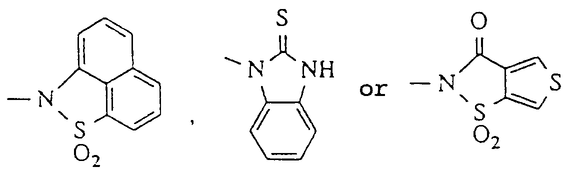 and
R⁹ denotes hydrogen, fluorine, chlorine, bromine, trifluoromethyl, hydroxyl or straight-chain or branched alkyl or alkoxy having, in each case, up to 4 carbon atoms,
optionally in an isomeric form,
and their salts.

3. Azaheterocylylmethyl-chromans according to Claim 1, wherein
A, B and D independently of one another
represent hydrogen, fluorine, chlorine, bromine, cyano, trifluoromethyl, trifluoromethoxy or hydroxyl, or
represent straight-chain or branched alkyl or alkenyl having, in each case, up to 4 carbon atoms, or
represent a group of the formula -NR¹R² or -OR⁵,
wherein
R¹ and R² are identical or different and denote hydrogen or straight-chain or branched alkyl having up to 4 carbon atoms, and
R⁵ denotes straight-chain or branched alkyl or alkenyl having up to 4 carbon atoms, which are optionally substituted by cyclopropyl or phenyl,
or
A has one of the abovementioned meanings
and
B and D together form a radical of the formula 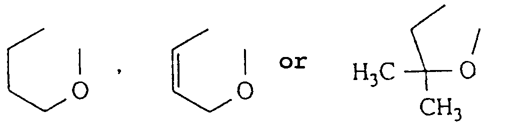
E represents a heterocyclic radical of the formula 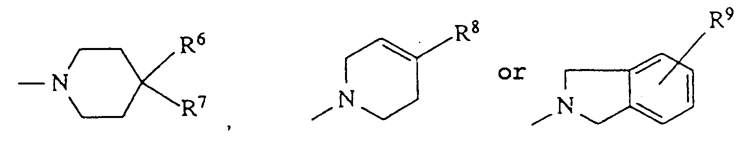 wherein
R⁶ denotes hydrogen, hydroxyl, fluorine or chlorine,
R⁷ and R⁸ independently of one another denote straight-chain or branched alkyl having up to 4 carbon atoms, which must be substituted by a radical of the formula 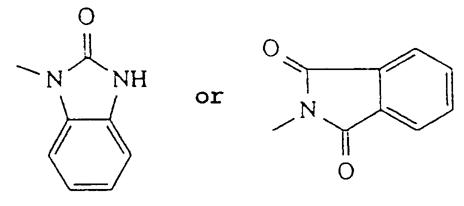 or denote a radical of the formula 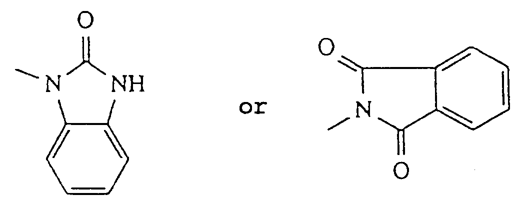 and
R⁹ denotes hydrogen, fluorine, chlorine, trifluoromethyl, hydroxyl, methyl, ethyl, methoxy or ethoxy,
optionally in an isomeric form,
and their salts.

4. Azaheterocylylmethyl-chromans according to Claims 1-3 for therapeutic use.

5. Process for the preparation of azaheterocylylmethyl-chromans according to Claims 1-3, characterised in that
[A] compounds of the general formula (II) 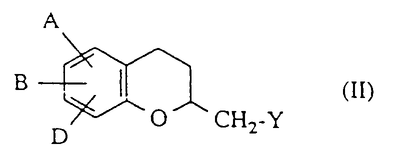 in which
A, B and D have the meaning mentioned in Claim 1
and
Y represents hydroxyl or
represents a typical leaving group, such as, for example, tosylate, chloride or mesylate, preferably tosylate,
are reacted directly with compounds of the general formula (III)
H-E (III),
in which
E has the meaning mentioned in Claim 1
in inert solvents, in the presence of a base and optionally of an auxiliary (catalyst, starter),
or
[B] in the case where R⁷ represents a radical of the formula -T-R¹⁰,
wherein
T denotes straight-chain or branched alkyl having up to 8 carbon atoms
and
R¹⁰ represents one of the heterocyclic radicals mentioned above under R⁷,
compounds of the general formula (IV), 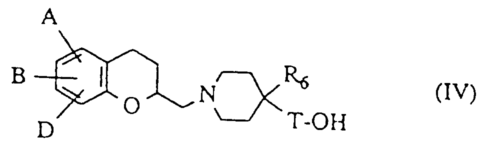 in which
A, B, D, R⁶ and T have the abovementioned meaning,
are reacted with compounds of the general formula (V)
H-R¹⁰ (V)
in which
R¹⁰ has the abovementioned meaning,
in inert solvents in a Mitsunobu reaction,
and, optionally, reductions are carried out by conventional methods, and
in the case of the enantiomers, either the compounds of the general formula (III) are reacted with compounds of the general formula (II) which are in the form of a single enantiomer, or the corresponding racemates of the compounds of the general formula (I) are separated by known methods for racemate separation, for example by separation via salts with acids in the form of a single enantiomer,
and, optionally, the substituents A, B and D are converted to derivatives, likewise by known methods.

6. Medicament containing at least one azaheterocyclylmethyl-chroman according to Claims 1-3.

7. Medicament according to Claim 6 for controlling diseases which are characterised by a disorder of the serotoninergic system.

8. Medicament according to Claim 6, for controlling diseases which are characterised by a disorder of the dopaminergic system.

9. Process for the preparation of medicaments according to Claim 6, characterised in that the azaheterocyclylmethyl-chromans are converted into a suitable administration form, optionally with the aid of conventional auxiliaries and excipients.

10. Use of the azaheterocyclyl-chromans according to Claims 1-3 for the preparation of medicaments.

## Revendications

1. Azahétérocyclylméthyl-chromanes de formule générale 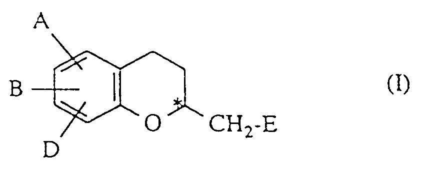 dans laquelle
A, B et D représentent, indépendamment les uns des autres, de l'hydrogène, un halogène, un groupe cyano, azido, nitro, difluorométhyle, trifluorométhyle, difluorométhoxy, trifluorométhoxy, hydroxy ou carboxy, ou bien
un groupe alkyle, alcényle, acyle ou alkoxycarbonyle linéaire ou ramifié ayant dans chaque cas jusqu'à 8 atomes de carbone, ou bien
un groupe de formule -NR¹R², -NR³-L-R⁴ ou -OR⁵, dans laquelle
R¹, R² et R³ sont identiques ou différents et représentent de l'hydrogène, un groupe alkyle linéaire ou ramifié ayant jusqu'à 8 atomes de carbone, phényle ou benzyle,
L est un groupe -CO- ou -SO₂-,
R⁴ est un groupe alkyle linéaire ou ramifié ayant jusqu'à 8 atomes de carbone ou un groupe benzyle, ou bien
un groupe aryle ayant 6 à 10 atomes de carbone, qui est substitué, le cas échéant, par un halogène, un radical hydroxy, nitro, cyano, trifluorométhyle, trifluorométhoxy ou par un radical alkyle ou alkoxy linéaire ou ramifié ayant jusqu'à 6 atomes de carbone,
R⁵ est un groupe alkyle ou un groupe alcényle linéaire ou ramifié ayant chacun jusqu'à 8 atomes de carbone et substitué, le cas échéant par un radical cycloalkyle de 3 à 6 atomes de carbone ou un radical phényle,
ou bien
A a l'une des définitions indiquées ci-dessus
et
B et D forment conjointement avec le noyau aromatique un carbocycle ou un hétérocycle pentagonal à heptagonal saturé, partiellement insaturé ou aromatique ayant jusqu'à 2 hétéro-atomes de la série S, N ou O, chacun de ces cycles pouvant posséder, le cas échéant, jusqu'à 2 fonctions carbonyle dans le noyau et étant substitué, le cas échéant, jusqu'à 2 fois identiques ou différentes par un radical alkyle, alcényle ou alkoxy linéaire ou ramifié ayant chacun jusqu'à 6 atomes de carbone, un radical hydroxy, cycloalkyle ayant 3 à 6 atomes de carbone, phényle, halogéno, cyano, nitro ou en configuration spiro par un reste de formule 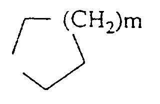 dans laquelle
m est le nombre 1 ou 2,
E représente un reste hétérocyclique de formule 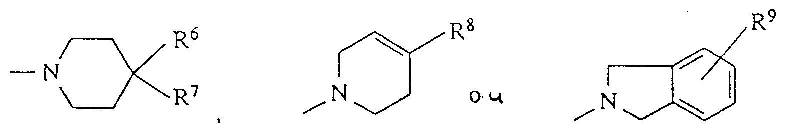 dans laquelle
R⁶ est de l'hydrogène, un radical hydroxy, un halogène ou un radical phényle,
R⁷ et R⁸ représentent, indépendamment l'un de l'autre,
un groupe alkyle linéaire ou ramifié ayant jusqu'à 8 atomes de carbone, qui doit être substitué par un reste de formule 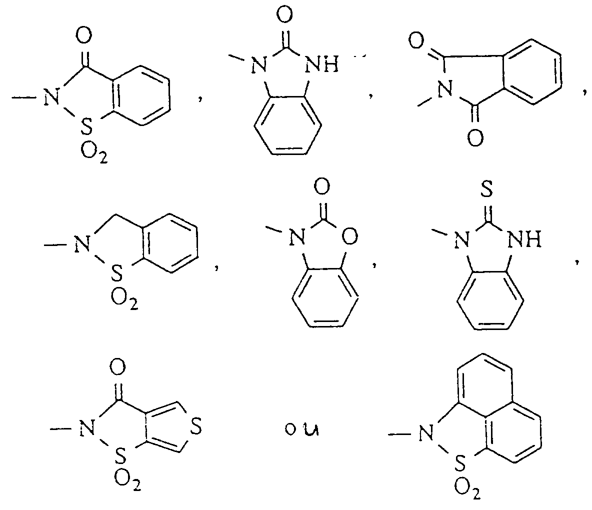 ou représentent un reste de formule 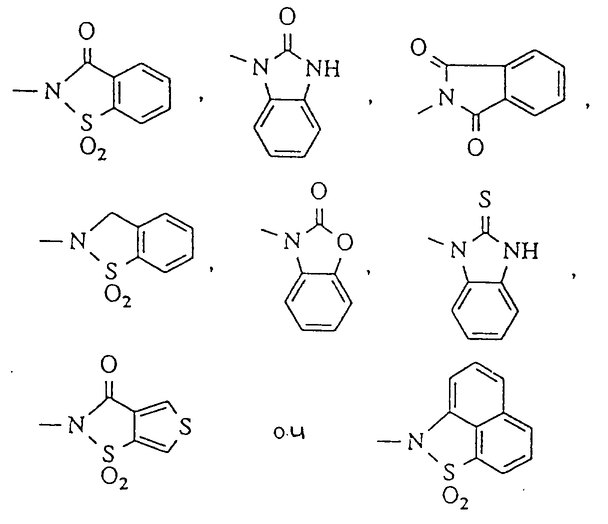 et
R⁹ est de l'hydrogène, un halogène, un groupe cyano, nitro, trifluorométhyle, trifluorométhoxy, hydroxy ou un groupe alkyle ou alkoxy linéaire ou ramifié ayant chacun jusqu'à 6 atomes de carbone,
le cas échéant sous une forme isomère,
et leurs sels.

2. Azahétérocyclylméthyl-chromanes suivant la revendication 1, dans lesquels
A, B et D représentent, indépendamment les uns des autres, de l'hydrogène, du fluor, du chlore, du brome, un groupe cyano, trifluorométhyle, difluorométhoxy, trifluorométhoxy ou hydroxy, ou
un groupe alkyle, alcényle, acyle ou alkoxycarbonyle linéaire ou ramifié ayant dans chaque cas jusqu'à 6 atomes de carbone, ou
un groupe de formule -NR¹R², -NR³-L-R⁴ ou -OR⁵, dans laquelle
R¹, R² et R³ sont identiques ou différents et représentent de l'hydrogène ou un groupe alkyle linéaire ou ramifié ayant jusqu'à 6 atomes de carbone,
L est un groupe -CO- ou -SO₂-,
R⁴ est un groupe alkyle linéaire ou ramifié ayant jusqu'à 6 atomes de carbone ou un groupe benzyle, ou représente
un groupe phényle qui est substitué, le cas échéant par du fluor, du chlore, du brome, un radical trifluorométhyle, trifluorométhoxy, hydroxy ou par un radical alkyle ou alkoxy linéaire ou ramifié ayant chacun jusqu'à 4 atomes de carbone,
R⁵ est un groupe alkyle ou alcényle linéaire ou ramifié ayant jusqu'à 6 atomes de carbone, chacun étant éventuellement substitué par un radical cyclopropyle, cyclopentyle, cyclohexyle ou phényle,
ou bien
A a l'une des définitions indiquées ci-dessus
et
B et D forment conjointement un reste de formule 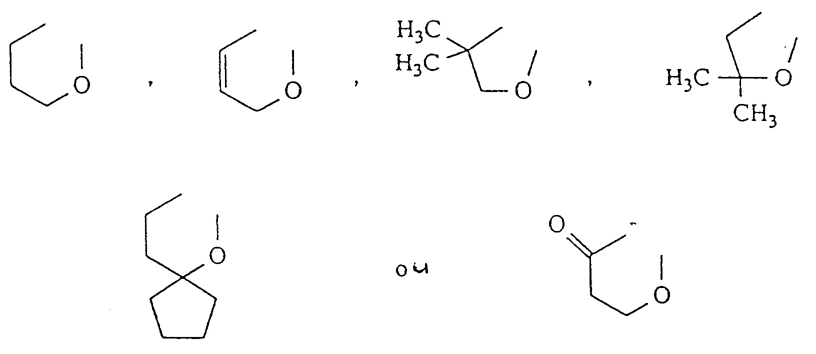
E représente un reste hétérocyclique de formule 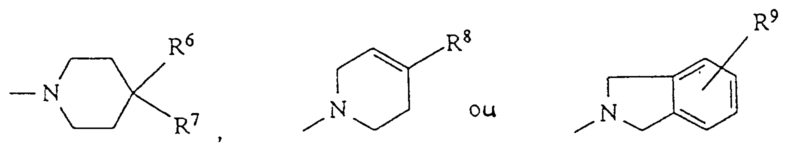 dans laquelle
R⁶ est de l'hydrogène, un radical hydroxy, du fluor, du chlore, du brome ou un radical phényle,
R⁷ et R⁸ représentent, indépendamment l'un de l'autre, un radical alkyle linéaire ou ramifié ayant jusqu'à 6 atomes de carbone, qui doit être substitué par un reste de formule 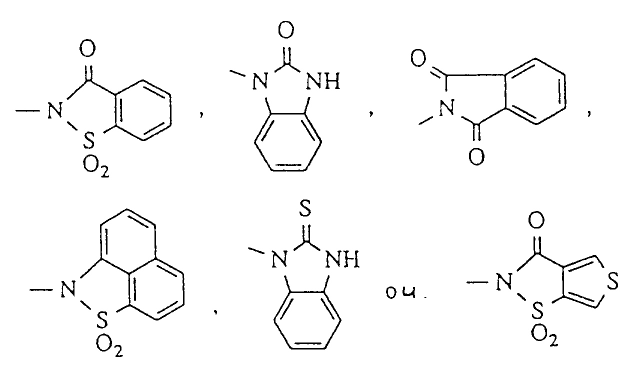 ou représentent un reste de formule 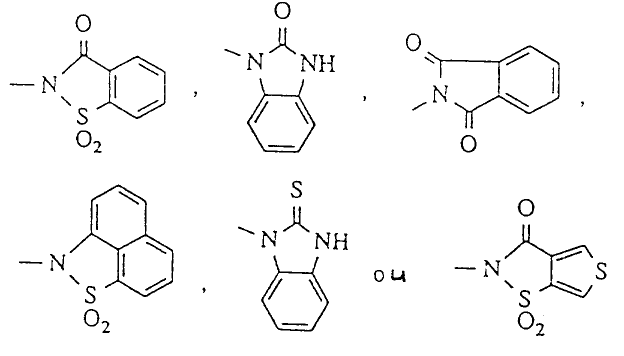 et
R⁹ est de l'hydrogène, du fluor, du chlore, du brome, un reste trifluorométhyle, hydroxy, alkyle ou alkoxy linéaire ou ramifié ayant chacun jusqu'à 4 atomes de carbone,
éventuellement sous une forme isomère, et leurs sels.

3. Azahétérocyclylméthyl-chromanes suivant la revendication 1, dans lesquels
A, B et D représentent, indépendamment les uns des autres, de l'hydrogène, du fluor, du chlore, du brome, un reste cyano, trifluorométhyle, trifluorométhoxy ou hydroxy, ou bien
un reste alkyle ou alcényle linéaire ou ramifié ayant chacun jusqu'à 4 atomes de carbone,
ou bien
un groupe de formule -NR¹R² ou -OR⁵,
dans laquelle
R¹ et R² sont identiques ou différents et représentent de l'hydrogène ou un reste alkyle linéaire ou ramifié ayant jusqu'à 4 atomes de carbone,
R⁵ est un reste alkyle ou alcényle linéaire ou ramifié ayant jusqu'à 4 atomes de carbone, ces restes étant éventuellement substitués par un radical cyclopropyle ou phényle,
ou bien
A a les définitions indiquées ci-dessus,
et
B et D forment ensemble un reste de formule 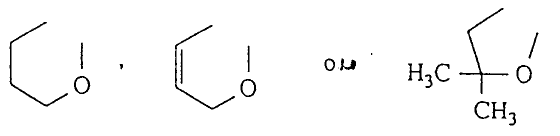
E est un reste hétérocyclique de formule 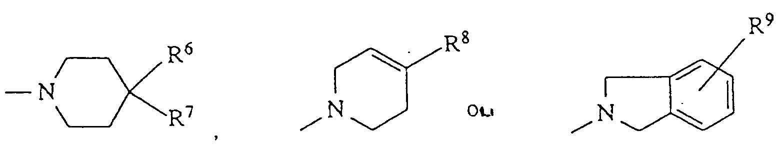 dans laquelle
R⁶ est de l'hydrogène, un radical hydroxy, du fluor ou du chlore,
R⁷ et R⁸ représentent, indépendamment l'un de l'autre, un groupe alkyle linéaire ou ramifié ayant jusqu'à 4 atomes de carbone, qui doit être substitué par un reste de formule 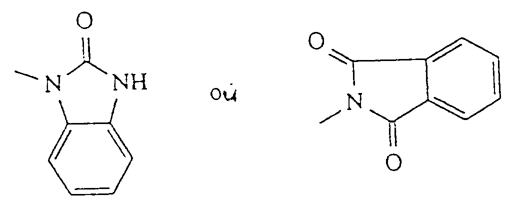 ou représentent un reste de formule 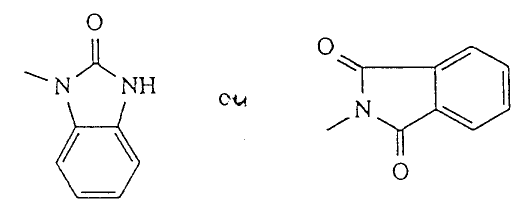 et
R⁹ est de l'hydrogène, du fluor, du chlore, un reste trifluorométhyle, hydroxy, méthyle, éthyle, méthoxy ou éthoxy,
le cas échéant sous une forme isomère, et leurs sels.

4. Azahétérocyclylméthyl-chromanes suivant les revendications 1 à 3, destinés à une application thérapeutique.

5. Procédé de production d'azahétérocyclylméthyl-chromanes suivant les revendications 1 à 3, caractérisé en ce que
[A] on fait réagir directement avec des composés de formule générale (III)
H-E (III)
dans laquelle
E a la définition indiquée dans la revendication 1,
des composés de formule générale (II) 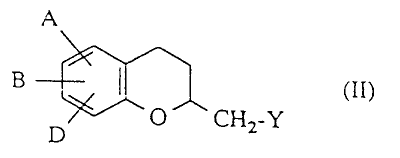 dans laquelle
A, B et D ont la définition indiquée dans la revendication 1 et
Y est un groupe hydroxy ou
un groupe partant typique, tel que par exemple tosylate, chlorure ou mésylate, de préférence un groupe tosylate,
dans des solvants inertes, en présence d'une base et, le cas échéant, d'une substance auxiliaire (catalyseur, agent d'amorçage),
ou bien
[B] au cas où R⁷ représente un reste de formule -T-R¹⁰,
dans laquelle
T est un groupe alkyle linéaire ou ramifié ayant jusqu'à 8 atomes de carbone
et
R¹⁰ représente l'un des restes hétérocycliques indiqués ci-dessus pour R⁷,
on fait réagir, dans une réaction de Mitsunobu, des composés de formule générale (IV) 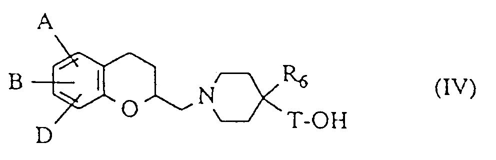 dans laquelle
A, B, D, R⁶ et T ont la définition indiquée ci-dessus,
avec des composés de formule générale (V)
H-R¹⁰ (V)
dans laquelle
R¹⁰ a la définition indiquée ci-dessus,
dans des solvants inertes,
et on conduit, le cas échéant, des réductions selon des modes opératoires classiques,
dans le cas des énantiomères, on fait réagir les composés de formule générale (III) avec des composés de pureté énantiomérique de formule générale (II), ou bien on sépare les racémates correspondants des composés de formule générale (I) par des opérations connues de dédoublement de racémates, par exemple par séparation en passant par des sels formés avec des acides de pureté énantiomérique,
et, le cas échéant, on dérivatise les substituants A, B et D, également par des opérations connues.

6. Médicament contenant au moins un azahétérocyclylméthyl-chromane suivant les revendications 1 à 3.

7. Médicament suivant la revendication 6, destiné à combattre des maladies qui sont caractérisées par un trouble du système sérotoninergique.

8. Médicament suivant la revendication 6, destiné à combattre des maladies qui sont caractérisées par un trouble du système dopaminergique.

9. Procédé de préparation de médicaments suivant la revendication 6, caractérisé en ce qu'on fait prendre une forme d'administration appropriée aux azahétérocyclylméthyl-chromanes, le cas échéant à l'aide de substances auxiliaires et de supports classiques.

10. Utilisation des azahétérocyclyl-chromanes suivant la revendication 1 à 3 pour la préparation de médicaments.
